**Europäisches Patentamt**

**European Patent Office**

**Office européen des brevets**

(19)

(11) Numéro de publication : **0 646 382 A1**

# (12) DEMANDE DE BREVET EUROPEEN

(21) Numéro de dépôt : **94402186.4**

(22) Date de dépôt : **29.09.94**

(51) Int. Cl.$^6$ : **A61M 5/168**

(30) Priorité : **30.09.93 FR 9311654**

(43) Date de publication de la demande :
**05.04.95 Bulletin 95/14**

(84) Etats contractants désignés :
**AT BE CH DE DK ES GB IT LI NL SE**

(71) Demandeur : **Becton Dickinson and Company**
**One Becton Drive**
**Franklin Lakes, New Jersey 07417-1880 (US)**

(72) Inventeur : **Jhuboo, Abdel-Nasser**
**47, avenue Général de Gaulle**
**F-38590 St. Etienne de St. Geoirs (FR)**
Inventeur : **Rebours, Pierre**
**Le Square B1 57 Avenue J. Jaurès**
**F-38500 Voiron (FR)**

(74) Mandataire : **Leszczynski, André**
**CABINET NONY & CIE.**
**29 rue Cambacérès**
**F-75008 Paris (FR)**

(54) **Procédé de détection d'occlusions dans une ligne de perfusion.**

(57) Procédé de détection d'occlusions dans une ligne de perfusion. Le procédé est mis en oeuvre dans une pompe à seringue motorisée (8) possédant un détecteur à jauges de contraintes et un contrôleur de microprocesseur. Les jauges de contraintes détectent la force sur le plongeur (18) de la seringue par rapport au temps. Le microprocesseur convertit la force en une pression et détermine le gradient de la relation pression/temps par rapport à un intervalle de temps particulier. Lorsque le gradient dépasse une valeur prédéterminée pendant une période de temps, le microprocesseur déclenche une alarme.

FIG-2

EP 0 646 382 A1

La présente invention a trait d'une manière générale à la détection d'occlusions dans une ligne de perfusion. En particulier, l'invention a trait à une pompe à seringue programmable, qui utilise un algorithme pour déterminer si une occlusion existe dans la ligne de perfusion.

Une pompe à seringue est un dispositif pour pomper un fluide depuis une seringue dans le corps d'un malade. La seringue est disposée dans la pompe et reliée au malade par l'intermédiaire d'une ligne de perfusion. Au cours de la perfusion d'une médication dans le corps d'un malade, il est possible qu'une occlusion se produise dans la ligne de perfusion. Un tel état peut provoquer une blessure au malade s'il n'est pas détecté.

Les pompes à seringue détectent généralement des occlusions en surveillant la pression sur le plongeur de la seringue afin de détecter des occlusions. Différents procédés sont utilisés afin d'effectuer ceci. Le procédé le plus simple implique la surveillance de la pression dans la ligne de perfusion à des intervalles de temps réguliers. Lorsque la pression dépasse une pression prédéterminée, un signal d'alarme est généré. Afin de minimiser des fausses alarmes, la pression à laquelle l'alarme est déclenchée est fixée à un niveau bien supérieur à la pression de ligne normale. L'utilisation de ce procédé exige un temps relativement long pour détecter une occlusion, puisque la pression dans la ligne doit atteindre le niveau d'alarme avant qu'une occlusion soit détectée.

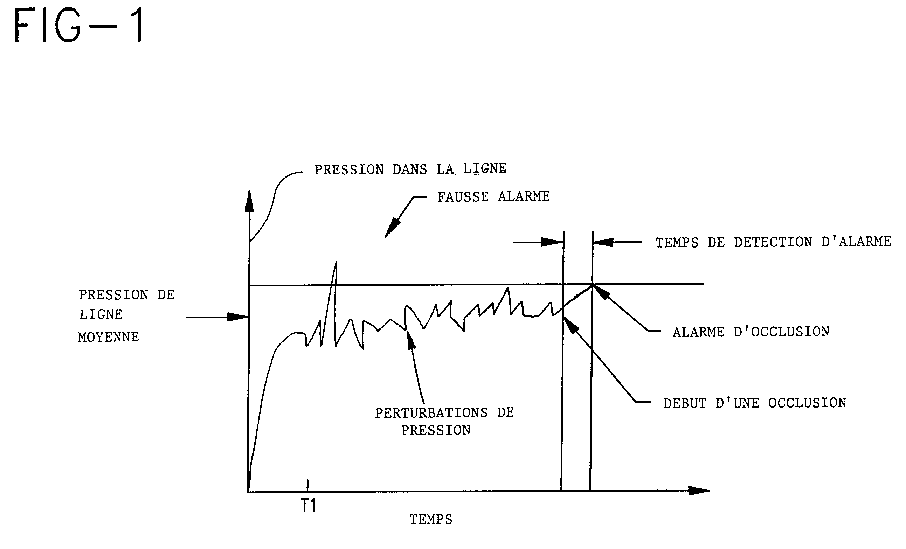

Selon un second procédé de l'art antérieur, la pression moyenne, plutôt que la pression instantanée dans la ligne de perfusion, est mesurée pendant une période de temps après un temps de stabilisation prédéterminé (cf. Figure 1). Le niveau d'alarme est fixé à un niveau proche de la pression de ligne moyenne. Ce procédé est susceptible de déclencher de fausses alarmes provoquées par de brefs accroissements de pression au-dessus de la limite d'alarme.

La présente invention a pour objet un procédé de détection d'occlusions dans une ligne de perfusion, le procédé étant caractérisé en ce qu'il comporte les étapes consistant à :

choisir une constante de gradient ;

mesurer une première pression dans la ligne de perfusion ;

mesurer une seconde pression dans la ligne de perfusion après un premier intervalle de temps ;

retrancher la première pression de la seconde pression pour obtenir une première différence de pression;

comparer la première différence de pression à la constante de gradient;

mesurer une troisième pression après un second intervalle de temps ;

retrancher la seconde pression de la troisième pression pour obtenir une seconde différence de pression;

comparer la seconde différence de pression à la constante de gradient ; et

générer un signal si la première différence de pression et la seconde différence de pression excèdent toutes deux la constante de gradient.

Selon d'autres caractéristiques avantageuses de l'invention:

- la constante de gradient est fonction du débit ;
- la constante de gradient est directement proportionnelle au débit ;
- les premier et second intervalles de temps sont sensiblement égaux ;
- le procédé comporte en outre une étape de modification automatique de l'intervalle de temps de façon inversement proportionnelle au débit de fluide dans la ligne de perfusion ;
- le procédé comporte une étape de réglage automatique de l'intervalle de temps en réponse à un bruit de la ligne de perfusion ;
- l'intervalle de temps est automatiquement accru pour réduire l'influence du bruit de la ligne de perfusion ;
- le procédé comporte en outre une étape de comparaison de la première différence de pression à une constante et de réglage du premier intervalle de temps si la première différence de pression excède la constante ;
- l'étape de réglage comporte l'accroissement du premier intervalle de temps ;
- la constante est déduite de la constante de gradient;
- la constante est la constante de gradient multipliée par un facteur de changement d'échelle ;
- le facteur de changement d'échelle est déduit empiriquement ;
- le procédé comporte une étape de comparaison de la première différence de pression à une constante et de réglage automatique de l'intervalle de temps si la première différence de pression est inférieure à la constante ;
- l'étape de réglage comporte une réduction de l'intervalle de temps ;
- le procédé comporte une étape d'optimalisation du premier intervalle de temps ;
- l'étape d'optimalisation met en oeuvre un procédé par dichotomie ;
- l'étape d'optimalisation comporte des étapes d'itération pour établir un intervalle de temps initial en comparant la première différence de temps à une constante ;
- l'étape d'optimalisation comporte des étapes d'accroissement de l'intervalle de temps si la valeur absolue de la première différence de pression dépasse une première constante et de diminution de l'intervalle de temps si la valeur absolue de la première différence de pression est inférieure à une seconde constante ;

- l'étape d'accroissement de l'intervalle de temps comporte le doublement du premier intervalle de temps ;
- l'étape de diminution de l'intervalle de temps comporte l'étape de réduction par deux du premier intervalle de temps.

La Figure 1 est un graphique de la pression en fonction du temps utilisant un procédé de l'art antérieur ;

la Figure 2 est une vue en perspective d'une pompe à seringue incorporant l'invention ;

la Figure 3 est une vue en perspective du mécanisme d'entraînement de la pompe à seringue ;

la Figure 4 est une vue en perspective du disque poussoir et du transducteur de force ;

la Figure 5 est un schéma synoptique des composants électroniques de l'invention ;

les Figures 6a à 6e sont des schémas des composants électroniques principaux de l'invention ;

la Figure 7 est un graphique de la pression en fonction du temps en utilisant la présente invention;

la Figure 8 est un organigramme illustrant le procédé de détection d'occlusions ;

la Figure 9 est un organigramme illustrant le procédé de détermination de l'intervalle de temps pour trouver le gradient de la courbe pression-temps; et

la Figure 10 est un organigramme représentant le procédé d'optimalisation de l'intervalle de temps pour trouver le gradient de la courbe pression-temps.

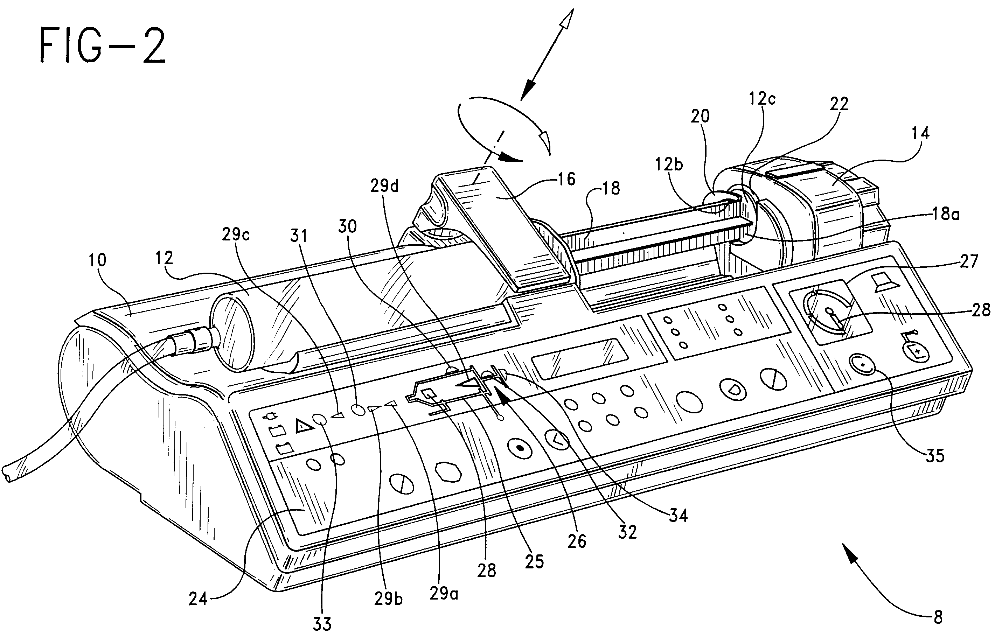

Une pompe à seringue 8 incorporant l'invention est représentée sur la Figure 2. Un boîtier 10 supporte une seringue 12, un poussoir 14 et une pince de seringue 16. La pince de seringue 16 maintient la seringue 12 en position sur le boîtier 10. Le plongeur 18 de la seringue 12 est poussé par un poussoir 14 qui est entraîné par un moteur électrique par l'intermédiaire d'une vis d'avance (cf. Figure 2).

Le poussoir 14 est muni d'un dispositif d'arrêt antisiphon 20 qui coopère avec un rebord 18a du plongeur 18, empêchant ainsi le plongeur 18 de se déplacer indépendamment du poussoir 14. Le poussoir 14 est également muni d'une plaque de pression 22 pour pousser directement contre le rebord 18a, pompant ainsi du fluide de la seringue 12.

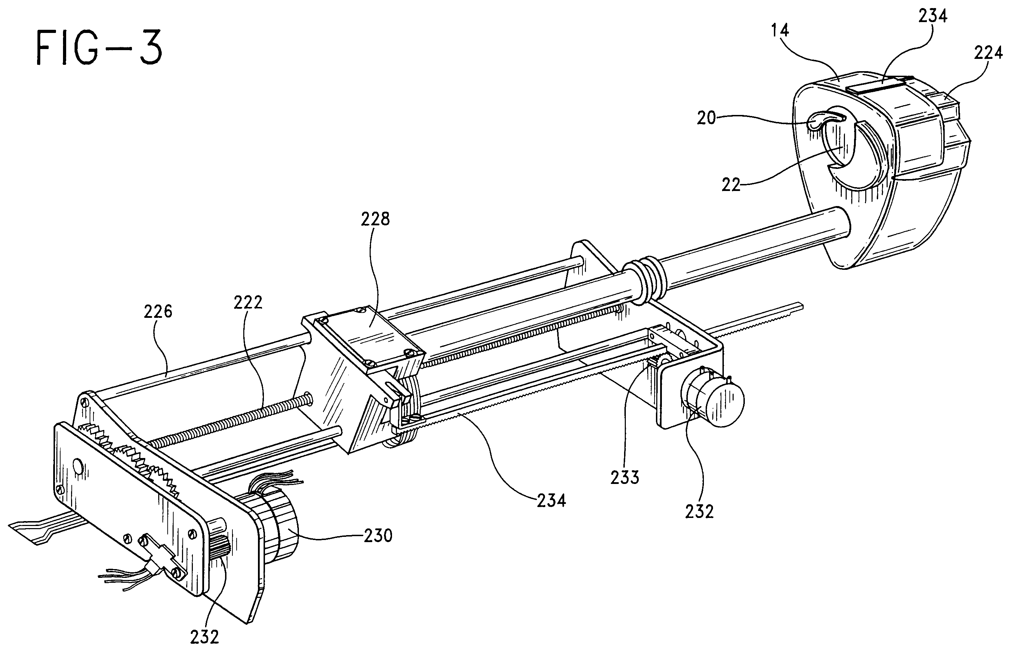

La Figure 3 représente le châssis et les constituants mécaniques de la pompe 8. Le châssis 226 porte un moteur 230 et une vis d'avance 222. Le moteur 230 entraîne la vis d'avance 222 par l'intermédiaire du train d'engrenages 232. Le poussoir 14 est entraîné par l'interaction du bloc poussoir 228 avec la vis d'avance 222. Le bloc poussoir contient des demi-écrous qui interagissent avec la vis d'avance 222.

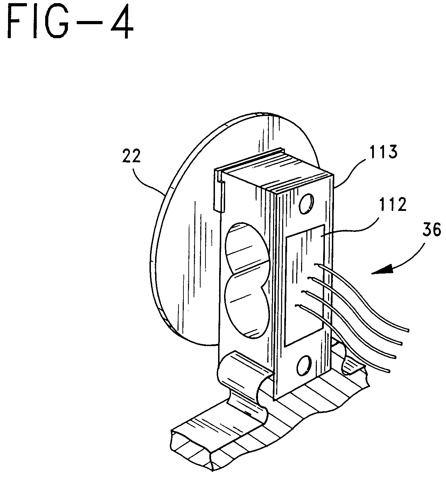

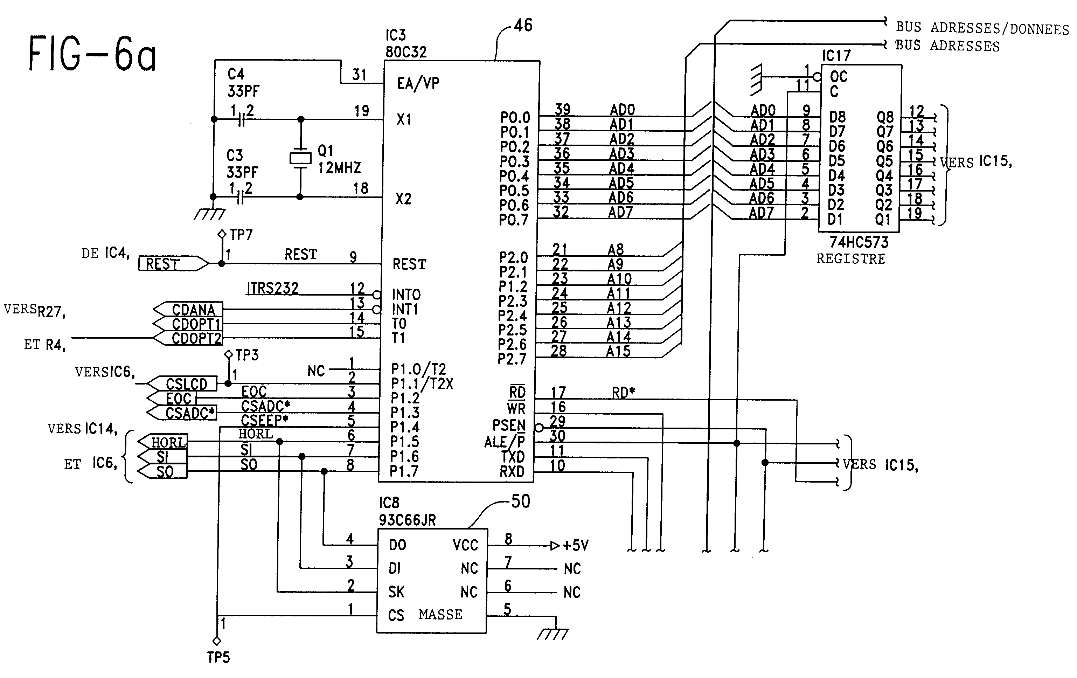

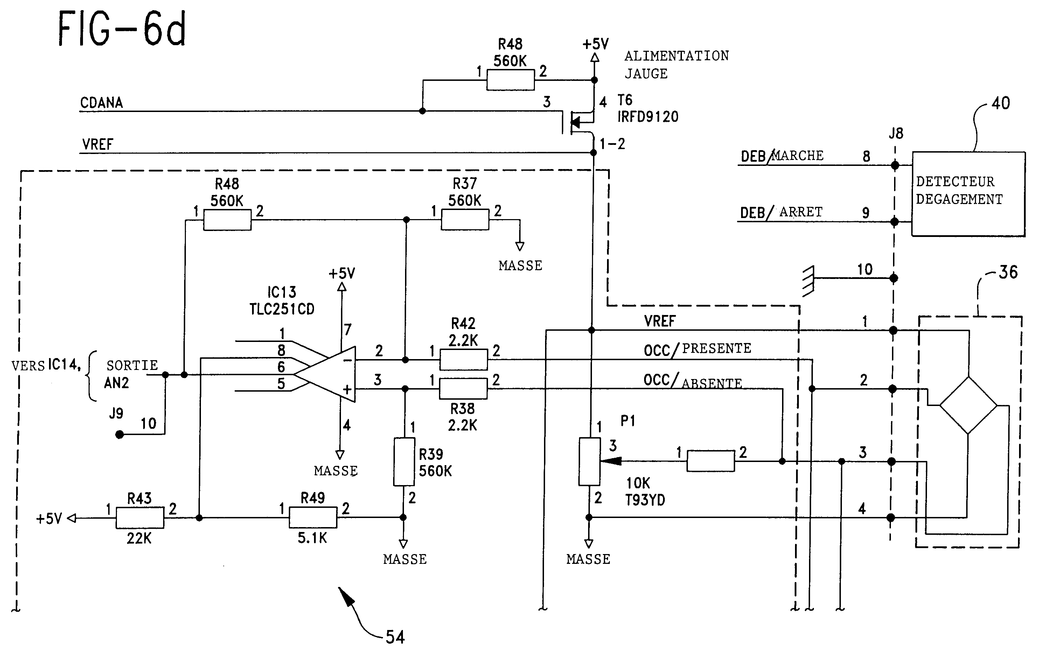

La Figure 4 représente plus en détail le transducteur de force 36. Le transducteur de force 36 est constitué de quatre jauges de contrainte montées en pont de Wheatstone. Le pont a une impédance de 350 ohms ou d'1 Kohm avec une tolérance de ±15%. La plage de mesures de force est de 0 à 150 N. La sensibilité du pont est 1,7 mV/V à 2,4 mV/V sous une charge de 150 N à 20°C. Le pont est alimenté par intermittence sous la commande d'un microprocesseur 46 (ligne CDANA sur les Figures 6a et 6d) afin d'économiser l'énergie.

Comme on le voit sur la Figure 4, les jauges de contrainte 112 sont collées sur un barreau 113. Lorsqu'une force est appliquée à la plaque de pression 22, le barreau 113 fléchit, provoquant une distorsion des jauges de contrainte 112 qui délivrent un signal de sortie 60.

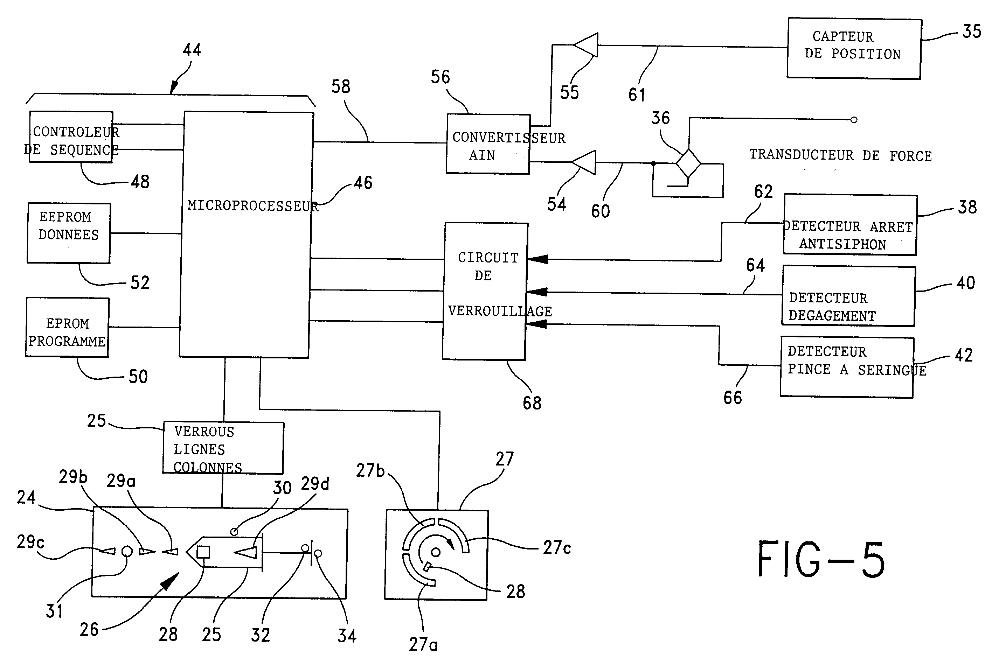

La Figure 5 est un schéma synoptique représentant les composants électroniques principaux de l'invention. Des transducteurs sont prévus pour détecter différents paramètres de la pompe à seringue qui sont affichés sur un panneau 24. Les transducteurs sont : le transducteur de force 36, le détecteur de dispositif d'arrêt antisiphon 38, le détecteur de dégagement 40 et le détecteur 42 de pince à seringue. Les signaux de sortie de ces transducteurs 60, 62, 64 et 66 respectivement sont appliqués à une unité de traitement centrale 44 par différents modules de traitement de signaux. Des schémas des différents modules électroniques sont représentés sur les Figures 6a-c. Les valeurs et les types des composants sont indiqués sur les schémas.

Le signal de sortie 60 du transducteur de force 36 est appliqué au module de conditionnement 54 (Figure 6d) et ensuite à un convertisseur analogique-numérique 56 qui convertit le signal de sortie conditionné du détecteur de force 36 en signal de sortie série 58. Le signal de sortie série 58 est ensuite appliqué à l'entrée 60 du microprocesseur 46.

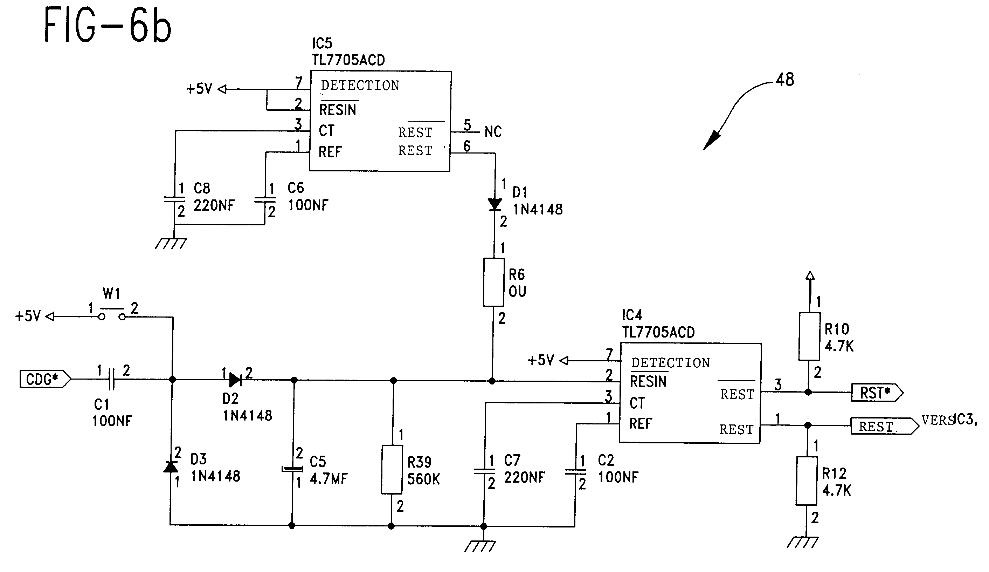

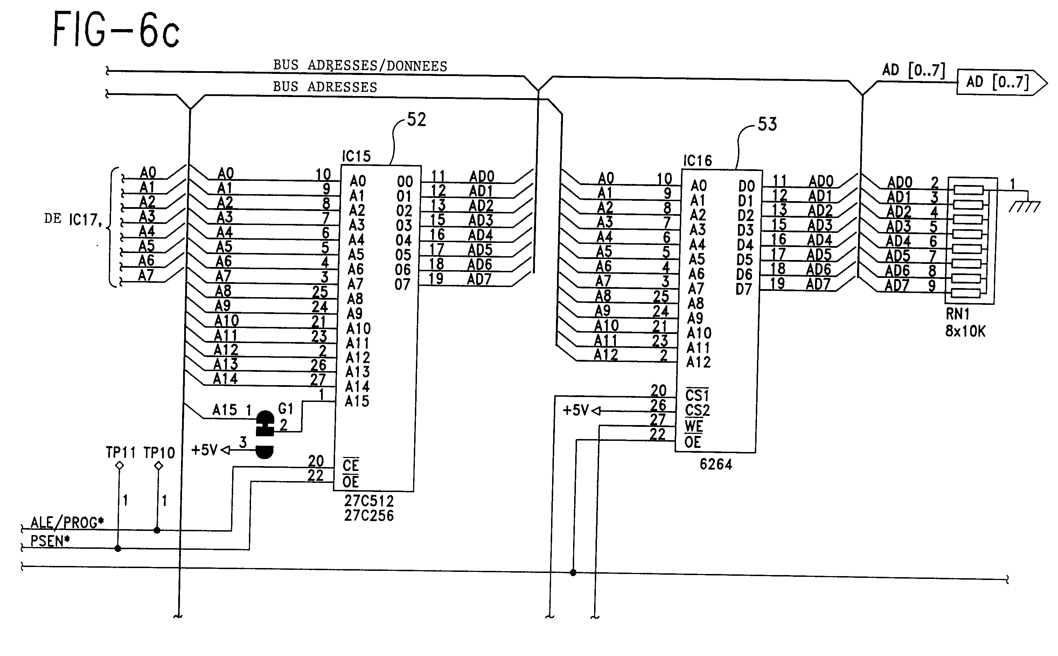

L'unité de traitement centrale 44 comporte le microprocesseur 46 (Figure 6a) avec une mémoire à accès sélectif 53 (Figure 6a), un contrôleur de séquence 48 (Figure 6b), une mémoire morte électriquement programmable 50 (Figure 6a) et une mémoire morte programmable effaçable électriquement 52 (Figure 6c). Le contrôleur de séquence 48 surveille le microprocesseur 46 pour assurer sa fonction correcte. La mémoire morte programmable effaçable électriquement 52 contient des données concernant les paramètres des seringues utilisées dans la pompe. La mémoire morte programmable électriquement 50 contient un programme logiciel qui commande le fonctionnement de la pompe à seringue.

Le signal de sortie du transducteur de force 36 est conditionné par un conditionnement de signal 54 (Figure 6d), qui convertit le signal de sortie du transducteur de force 36 en une forme appropriée pour une application à un convertisseur analogique-numérique 56 (Figure 6e). Le convertisseur analogique-numérique 56 numérise le signal de sortie analogique et délivre un signal de sortie série 58 qui est à son tour appliqué au point de connexion d'entrée 60 du microprocesseur 46.

Dans la mémoire morte électriquement programmable 50 se trouve un programme logiciel pour le microprocesseur 46 qui calcule la pression à l'intérieur de la seringue 12 continûment car la force sur le plongeur 18 est mesurée par le transducteur de force 36. Certains paramètres qui sont utilisés par le programme pour calculer la pression dans la seringue sont mémorisés dans la mémoire morte programmable effaçable électriquement 52. Puisque la pompe à seringue 8 est programmable pour recevoir différents types de seringues, un ensemble de paramètres pour chaque type de seringue, est mémorisé dans la mémoire morte programmable effaçable électriquement 52.

Les paramètres mémorisés dans la mémoire morte programmable effaçable électriquement 52 comprennent :

Ff = force de frottement moyenne entre le plongeur de la seringue et le fût de la seringue à pression nulle (atmosphérique).

Pc = la pression dans la seringue lorsqu'une force d'étalonnage est appliquée au plongeur. La force d'étalonnage est typiquement 50 N et conduit à une valeur de Pc d'environ 0,7 bar, un seuil de pression usuel.

Fc = la force avec laquelle le plongeur est chargé pour obtenir une pression de Pc dans la seringue.

Le programme dans la mémoire morte électriquement programmable 50 est utilisé par le microprocesseur 46 pour calculer la pression dans la seringue. Le microprocesseur 46 compare ensuite la pression calculée à une valeur de pression parmi les valeurs mémorisées dans la mémoire morte programmable effaçable électriquement 52 pour cette seringue. Si la pression calculée dépasse la pression mémorisée, une alarme d'occlusion est générée par le microprocesseur 46.

L'algorithme pour calculer la pression dans la seringue est:

$$P = \frac{(F - Ff)}{Fc - Ff} \cdot Pc$$

où F est la force mesurée par le transducteur de force 36 et Fc, Ff et Pc sont les paramètres décrits ci-dessus.

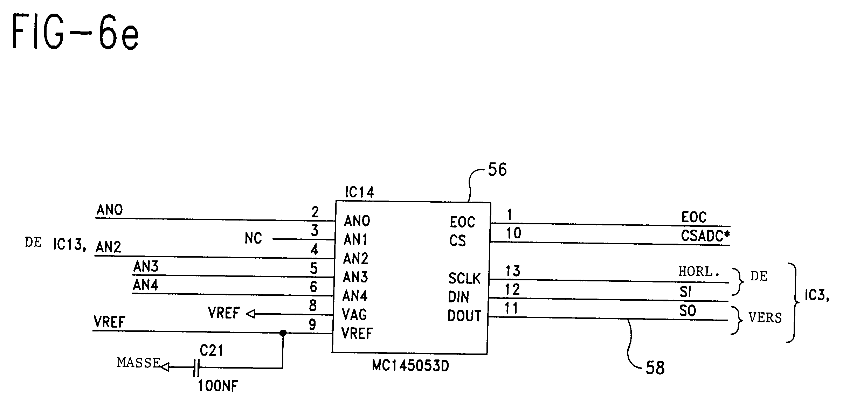

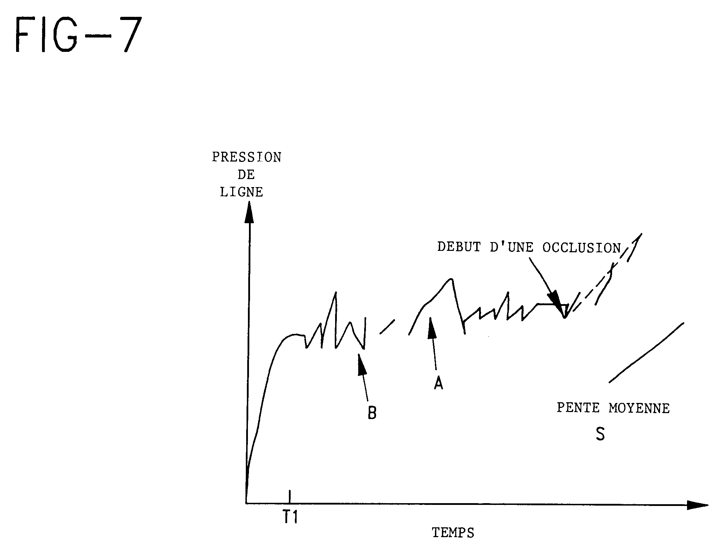

Si une occlusion se produit dans le trajet de fluide, la pression dans le trajet de fluide augmentera. Si le moteur 230 continue à entraîner le poussoir 14, la pression dans le trajet de fluide augmentera avec le temps. Un graphique de la pression dans la ligne de fluide en fonction du temps est représenté sur la Figure 7. Au cours de la perfusion, la pression dans la ligne de fluide peut subir des perturbations qui ne sont pas provoquées pas une occlusion. Par exemple, puisque la force de frottement entre la butée de la seringue 12 et la paroi intérieure de la seringue varie non linéairement, une perturbation de pression (indiquée "A" sur la Figure 7) peut se produire. Des perturbations peuvent également être provoquées par la présence d'autres dispositifs de perfusion sur la ligne. Une telle perturbation est indiquée par "B" sur la Figure 7.

Une occlusion est détectée en déterminant le gradient "S" de la courbe pression/temps représentée sur la Figure 7 et en le comparant à un gradient prédéterminé. La pression dans la ligne de fluide est mesurée de manière décrite ci-dessus.

Les mesures de pression suivantes sont effectuées aux intervalles de temps suivants :

$P_i$ = la pression moyenne dans l'intervalle
$[t_i ; t_i + \Delta t]$

$P_{i+1}$ = la pression moyenne durant l'intervalle
$[t_i + \Delta t ; t_i + 2 \Delta t]$

$P_{i+2}$ = la pression moyenne durant l'intervalle
$[t_i + 2 \Delta t ; t_i + 3 \Delta t]$

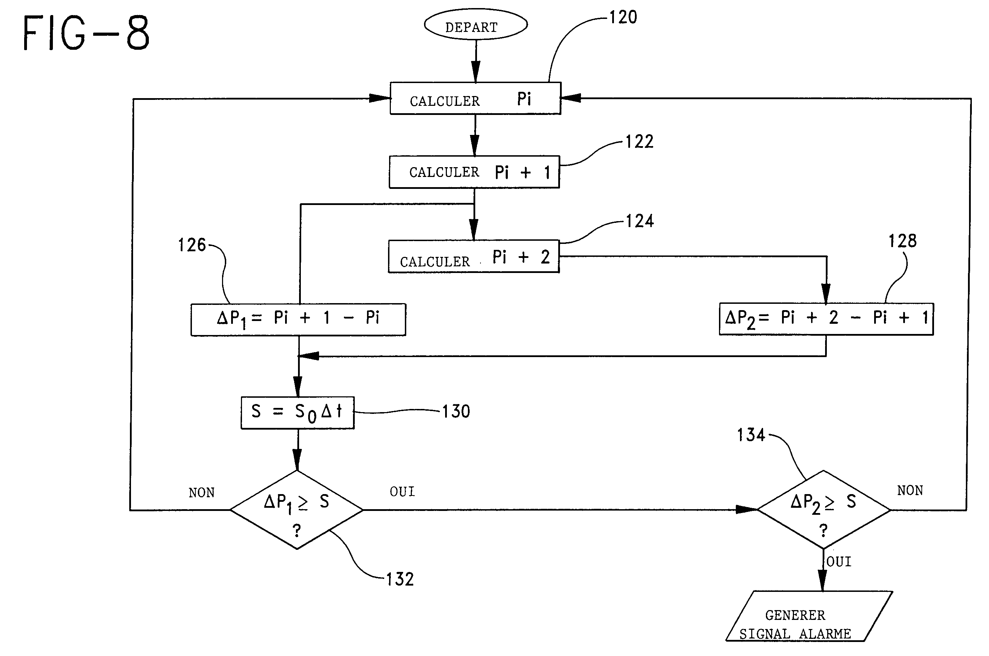

Ces étapes sont représentées en tant qu'étapes 120, 122, 124 dans l'organigramme de la Figure 8.

Le gradient de la courbe pression/temps de la Figure 7 peut être défini d'une manière générale par :

$$S = \Delta P / \Delta t$$

où $\Delta P$ est l'accroissement de pression dans la ligne de perfusion dans un intervalle de temps donné $\Delta t$. L'amplitude de S est proportionnelle au débit du liquide dans la ligne de fluide.

La mesure de gradient est réalisée comme suit. Le microprocesseur 46 est programmé pour calculer la différence entre des pressions à la fin d'intervalles de temps successifs et la compare ensuite à une constante déduite d'un nombre représentatif d'un gradient acceptable prédéterminé de la courbe pression/temps ($S_0$) et de l'amplitude de l'intervalle de temps $\Delta t$.

Le microprocesseur retranche ainsi $P_i$ de $P_{i+1}$ et compare le résultat à $S_0 \Delta t$. Cf. les étapes 126, 130, 132 de la Figure 6. Si $\Delta P_1 = P_{i+1} - P_i$ est supérieur à $S_0 \Delta t$, le gradient de la courbe pression/temps est supérieur à un niveau admissible. Ceci signifie à son tour qu'il existe une occlusion dans la ligne de fluide et un signal d'alarme est généré pour allumer un voyant 29b (étape 134 sur la Figure 8). Une autre comparaison peut être effectuée en retranchant $P_{i+1}$ de $P_{i+2}$ et en comparant le résultat $\Delta P_2$ à $S_0 \Delta t$ (étapes 128, 130 et 134). Il est possible d'utiliser uniquement la première comparaison ("si $\Delta P_1 \geqq S_0 \Delta t$") ou, de préférence, la première en combinaison avec la seconde comparaison ("si $\Delta P_2 \geqq S_0 \Delta t$"). C'est-à-dire qu'un signal d'occlusion sera généré uniquement si $\Delta P_1$ et $\Delta P_2$ sont tous deux supérieurs à ou égaux à $S_0 \Delta t$.

$S_0$ est déduit expérimentalement en mesurant les gradients de la courbe pression/temps avec des occlusions se trouvant dans la ligne de fluide et différents débits. $S_0$ est directement proportionnel au débit. Le microprocesseur 46 est programmé pour permettre à différentes valeurs de $S_0$ d'être choisies avant ou durant le processus de perfusion. En utilisant le procédé décrit ci-dessus, même si $S_0$ est une faible valeur, il est possible de détecter une occlusion partielle du fait que c'est la vitesse de variation de pression plutôt que la pression elle-même qui est mesurée.

L'intervalle de temps $\Delta t$ est réglé automatiquement durant une perfusion. L'intervalle de temps $\Delta t$ est inversement proportionnel au débit r. Le débit r peut être choisi par l'utilisateur et programmé dans la pompe à seringue 8. La grandeur de $\Delta t$ est également fonction de la quantité de bruit dans le système. Si $\Delta t$ est faible, le système sera sensible à des variations rapides de pression.

Si le système comporte du bruit, $\Delta t$ doit être important et le système sera moins sensible à des variations de pression. Si le système est moins sujet à des bruits, $\Delta t$ peut être faible, faisant réagir le système plus rapidement à une occlusion. Ci-après est donnée une description du procédé d'optimisation de $\Delta t$, le temps pendant lequel le gradient de la courbe pression/temps est mesurée.

La valeur initiale de $\Delta t$, $\Delta t_0$ est inversement proportionnelle à la vitesse de perfusion r. Par exemple, pour r = 1 ml par heure, $\Delta t_0$ = 1 minute. La valeur de $\Delta t0$ est déterminée empiriquement. Ayant choisi une valeur initiale pour $\Delta t$, il faut tout d'abord établir si $\Delta t$ doit être augmenté ou diminué.

On considère l'intervalle de temps $[t_i, t_i+\Delta t]$, si $\Delta P_1 > K_1 S_0 \Delta t$, alors $\Delta t$ doit être augmenté puisque la pression augmente rapidement et que le système sera susceptible d'être sujet à un bruit.
si $\Delta P_1 < K_2 S_0 \Delta t$, alors $\Delta t$ doit être diminué puisque la pression augmente moins rapidement et que le système sera moins sujet à un bruit.

Dans le mode de réalisation préféré, les constantes $K_1$ et $K_2$ sont les suivantes : $K_1$ = 1/4 ; $K_2$ = 1/8 et sont déduites empiriquement.

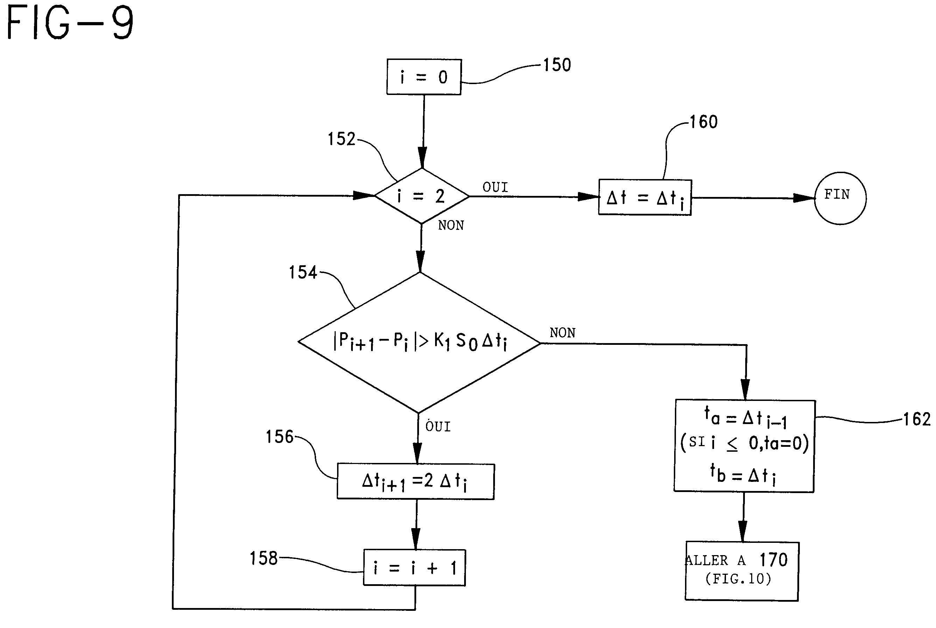

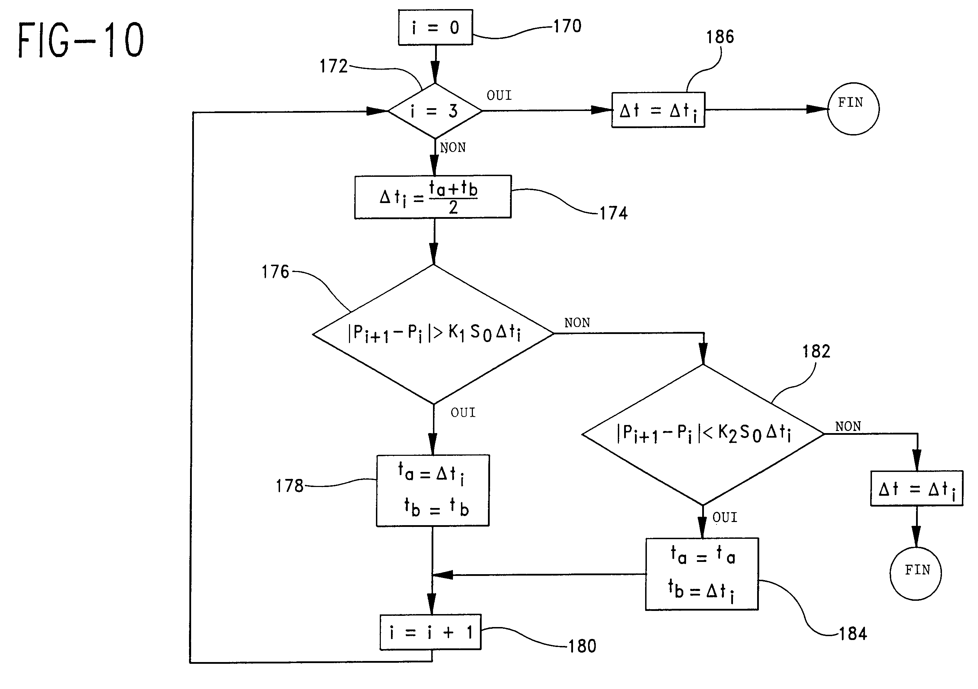

La valeur optimale pour $\Delta t$ est atteinte par application de l'algorithme représenté sur les Figures 9 et 10. Initialement, l'intervalle dans lequel se trouve $\Delta t$ est établi au moyen de l'algorithme représenté sur la Figure 9. Il a été constaté expérimentalement que seulement trois itérations sont nécessaires pour établir l'intervalle.

Un compteur "i" est tout d'abord mis à zéro (étape 150 sur la Figure 9). Une valeur initiale de $\Delta t$ est établie dans le mode de réalisation préféré, ($\Delta t_0$= 1 minute). Si moins de deux itérations ont été effectuées (c'est-à-dire i$\neq$2 étape 152), la variation de pression $\Delta P$ (c'est-à-dire $P_{i+1} - P_i$) dans un intervalle $\Delta t$ entre $t_i$ et $t_{i+1}$ est déterminée. La valeur absolue de la différence de pression est comparée à $K_1 S_0 \Delta t_i$, en utilisant les valeurs déterminées ci-dessus (étape 154). Si la valeur absolue de $\Delta P_i$ est supérieure à $K_1 S_0 \Delta t_i$, alors $\Delta t$ pour le temps suivant, $\Delta t_{i+1}$ est doublé (c'est-à-dire $\Delta t_{i+1} = 2\Delta t_i$) (étape 156) et le compteur "i" est augmenté de 1 (étape 158), en prenant l'algorithme pour l'intervalle de temps suivant. Après trois itérations (c'est-à-dire i=2), la valeur de $\Delta t_i$ est affectée à $\Delta t$ (étape 160) puisque l'intervalle de temps est suffisamment grand. Si la valeur absolue de $\Delta P$ n'est pas supérieure à $K_1 S_0 \Delta t$, alors un intervalle de temps $[t_a ; t_b]$ est déterminé en affectant des valeurs comme suit (étape 162) ;

$$t_a = \Delta t_{i-1}$$
$$t_a = 0 \text{ si } i = 0$$
$$t_b = \Delta t_i$$

Le système progresse ensuite pour optimaliser $\Delta t$ pour de faibles intervalles de temps selon un procédé appelé "procédé par dichotomie" représenté sur la Figure 3.

Un compteur "i" est mis à zéro (étape 170). Empiriquement, on constate qu'un maximum de quatre itérations sont nécessaires pour obtenir une optimalisation satisfaisante de $\Delta t$ indépendamment du débit. Si moins de quatre itérations ont été effectuées (étape 172), $\Delta t$ pour un intervalle particulier est fixé à $(t_a + t_b)/2$ (c'est-à-dire la moyenne de l'intervalle établi ci-dessus (étape 174)). Si la valeur absolue de $\Delta P(P_{i+1} - P_i)$ pour cet intervalle est supérieure à $K_1 S_0 \Delta t_i$ (étape 176), l'intervalle est affecté de nouveau comme suit (étape 178):

$$t_a = \Delta t_i$$
$$t_b = t_b$$

et le compteur est augmenté de 1 (étape 180). Le processus continue l'itération jusqu'à ce que quatre itérations soient effectuées.

Si la valeur absolue de $\Delta P$ dans un intervalle quelconque n'est pas supérieure à $K_1 S_0 \Delta t_i$, alors cette valeur est comparée à $K_2 S_0 \Delta t_i$ (étape 182). Si la valeur absolue de $\Delta P$ est inférieure à $K_2 S_0 \Delta t_i$, alors l'intervalle est à nouveau déterminé comme suit (étape 184) :

$$t_a = t_a$$
$$t_b = \Delta t_i$$

Le compteur est augmenté (étape 180) et le processus d'itération est poursuivit.

Si la valeur absolue de $\Delta P$ n'est pas inférieure à $K_2 S_0 \Delta t_i$, alors $\Delta t$ a été optimalisé et l'intervalle de temps $\Delta t$ devient $\Delta t_i$. L'algorithme s'arrête alors.

Dans un cas quelconque, après quatre itérations (c'est-à-dire i=3), $\Delta t$ devient $\Delta t_i$ (étape 186) et l'algorithme s'arrête.

## Revendications

1. Procédé de détection d'occlusions dans une ligne de perfusion, caractérisé en ce qu'il comporte les étapes consistant à :

    choisir une constante de gradient ;

    mesurer une première pression dans la ligne de perfusion ;

    mesurer une seconde pression dans la ligne de perfusion après un premier intervalle de temps ;

retrancher la première pression de la seconde pression pour obtenir une première différence de pression ;

comparer la première différence de pression à la constante de gradient ;

mesurer une troisième pression après un second intervalle de temps ;

retrancher la seconde pression de la troisième pression pour obtenir une seconde différence de pression ;

comparer la seconde différence de pression à la constante de gradient ; et

générer un signal si la première différence de pression et la seconde différence de pression excèdent toutes deux la constante de gradient.

2. Procédé selon la revendication 1, caractérisé en ce que la constante de gradient est fonction du débit.

3. Procédé selon la revendication 1 ou 2, caractérisé en ce que la constante de gradient est directement proportionnelle au débit.

4. Procédé selon la revendication 1, caractérisé en ce que les premier et second intervalles de temps sont sensiblement égaux.

5. Procédé selon la revendication 1, caractérisé en ce qu'il comporte en outre une étape de modification automatique de l'intervalle de temps en proportion inverse du débit de fluide dans la ligne de perfusion.

6. Procédé selon la revendication 1, caractérisé en ce qu'il comporte en outre une étape de réglage automatique de l'intervalle de temps en réponse à un bruit de la ligne de perfusion.

7. Procédé selon la revendication 6, caractérisé en ce que l'intervalle de temps est automatiquement augmenté pour réduire l'influence du bruit de la ligne de perfusion.

8. Procédé selon la revendication 1, caractérisé en ce qu'il comporte en outre une étape de comparaison de la première différence de pression à une constante et de réglage du premier intervalle de temps si la première différence de pression excède la constante.

9. Procédé selon la revendication 8, caractérisé en ce que l'étape de réglage comporte l'accroissement du premier intervalle de temps.

10. Procédé selon la revendication 8, caractérisé en ce que la constante est déduite de la constante de gradient.

11. Procédé selon la revendication 8, caractérisé en ce que la constante est la constante de gradient multipliée par un facteur de changement d'échelle.

12. Procédé selon la revendication 11, caractérisé en ce que le facteur de changement d'échelle est déduit empiriquement.

13. Procédé selon la revendication 1, caractérisé en ce qu'il comporte en outre une étape de comparaison de la première différence de pression à une constante et de réglage automatique de l'intervalle de temps si la première différence de pression est inférieure à la constante.

14. Procédé selon la revendication 13, caractérisé en ce que l'étape de réglage comporte la réduction de l'intervalle de temps.

15. Procédé selon la revendication 1, caractérisé en ce qu'il comporte en outre une étape d'optimalisation du premier intervalle de temps.

16. Procédé selon la revendication 15, caractérisé en ce que l'étape d'optimalisation met en oeuvre un procédé par dichotomie.

17. Procédé selon la revendication 16, caractérisé en ce que l'étape d'optimalisation comporte des étapes d'itération pour établir un intervalle de temps initial en comparant la première différence de pression à une constante.

18. Procédé selon la revendication 16, caractérisé en ce que l'étape d'optimalisation comporte des étapes d'accroissement de l'intervalle de temps si la valeur absolue de la première différence de pression dépasse une première constante et de diminution de l'intervalle de temps si la valeur absolue de la première différence de pression est inférieure à une seconde constante.

19. Procédé selon la revendication 18, caractérisé en ce que l'étape d'accroissement de l'intervalle de temps comporte le doublement du premier intervalle de temps.

20. Procédé selon la revendication 18, caractérisé en ce que l'étape de réduction de l'intervalle de temps comporte l'étape de division par deux du premier intervalle de temps.

# FIG-1

PRESSION DANS LA LIGNE

FAUSSE ALARME

TEMPS DE DETECTION D'ALARME

PRESSION DE
LIGNE
MOYENNE

ALARME D'OCCLUSION

DEBUT D'UNE OCCLUSION

PERTURBATIONS DE
PRESSION

T1

TEMPS

EP 0 646 382 A1

FIG-2

EP 0 646 382 A1

FIG-3

FIG-4

FIG-5

CONTROLEUR DE SEQUENCE — 48

EEPROM DONNEES — 52

EPROM PROGRAMME — 50

MICROPROCESSEUR — 46

44

58

CONVERTISSEUR AIN — 56

55

61

CAPTEUR DE POSITION — 35

36

54   60   TRANSDUCTEUR DE FORCE

62   DETECTEUR ARRET ANTISIPHON — 38

CIRCUIT DE VERROUILLAGE

64   DETECTEUR DEGAGEMENT — 40

66   DETECTEUR PINCE A SERINGUE — 42

68

VERROUS LIGNES COLONNES

25

29b  29a  30  29d

24

29c

31  26  28  25  32  34

27b  27

27c

27a  28

EP 0 646 382 A1

# FIG-6a

BUS ADRESSES/DONNEES
BUS ADRESSES

IC3
80C32

IC17
74HC573
REGISTRE

VERS IC15,

IC8
93C66JR

46

50

EP 0 646 382 A1

FIG-6b

48

IC5
TL7705ACD

DETECTION
RESIN
CT    REF
REST
REST

NC

+5V

C6 100NF
C8 220NF

D1 1N4148
R6 OU

IC4
TL7705ACD

DETECTION
RESIN
CT    REF
REST
REST

+5V

C7 220NF
C2 100NF

R39 560K
C5 4.7MF

D2 1N4148
D3 1N4148

R10 4.7K
RST*

R12 4.7K
REST
VERSC3,

W1

+5V

C1 100NF
CDG*

# FIG-6c

BUS ADRESSES/DONNEES

BUS ADRESSES

AD [0..7]    AD [0..7]

52    53

IC15    IC16

| | | | IC15 | | | | | |
|---|---|---|---|---|---|---|---|---|---|

DE IC17,

| A0 | A0 | 10 | A0 | 00 | 11 | AD0 |
|---|---|---|---|---|---|---|
| A1 | A1 | 9 | A1 | 01 | 12 | AD1 |
| A2 | A2 | 8 | A2 | 02 | 13 | AD2 |
| A3 | A3 | 7 | A3 | 03 | 15 | AD3 |
| A4 | A4 | 6 | A4 | 04 | 16 | AD4 |
| A5 | A5 | 5 | A5 | 05 | 17 | AD5 |
| A6 | A6 | 4 | A6 | 06 | 18 | AD6 |
| A7 | A7 | 3 | A7 | 07 | 19 | AD7 |
| | A8 | 25 | A8 | | | |
| | A9 | 24 | A9 | | | |
| | A10 | 21 | A10 | | | |
| | A11 | 23 | A11 | | | |
| | A12 | 2 | A12 | | | |
| | A13 | 26 | A13 | | | |
| | A14 | 27 | A14 | | | |
| | | 1 | A15 | | | |

A15 1    G1
         2

+5V    3

TP11 TP10

1    1

ALE/PROG*

PSEN*

20    CE
22    OE

27C512
27C256

IC16

| A0 | 10 | A0 | D0 | 11 | AD0 |
|---|---|---|---|---|---|
| A1 | 9 | A1 | D1 | 12 | AD1 |
| A2 | 8 | A2 | D2 | 13 | AD2 |
| A3 | 7 | A3 | D3 | 15 | AD3 |
| A4 | 6 | A4 | D4 | 16 | AD4 |
| A5 | 5 | A5 | D5 | 17 | AD5 |
| A6 | 4 | A6 | D6 | 18 | AD6 |
| A7 | 3 | A7 | D7 | 19 | AD7 |
| A8 | 25 | A8 | | | |
| A9 | 24 | A9 | | | |
| A10 | 21 | A10 | | | |
| A11 | 23 | A11 | | | |
| A12 | 2 | A12 | | | |

+5V    20    CS1
       26    CS2
       27    WE
       22    OE

6264

| AD0 | 2 |
|---|---|
| AD1 | 3 |
| AD2 | 4 |
| AD3 | 5 |
| AD4 | 6 |
| AD5 | 7 |
| AD6 | 8 |
| AD7 | 9 |

1

RN1
8x10K

EP 0 646 382 A1

# FIG-6d

# FIG-6e

EP 0 646 382 A1

# FIG-7

PRESSION
DE
LIGNE

DEBUT D'UNE OCCLUSION

B

A

PENTE MOYENNE
S

T1

TEMPS

EP 0 646 382 A1

# FIG-8

```
                    ( DEPART )────────────── 120

              ┌──────────────────────┐
        ──────►│  CALCULER    Pi      │◄──────────────────
        │      └──────────────────────┘                  │
        │                 │                               │
        │      ┌──────────────────────┐                  │
        │      │  CALCULER  Pi + 1    │────── 122         │
        │      └──────────────────────┘                  │
        │           │        │                            │
        │           │   ┌──────────────────────┐          │
        │           │   │  CALCULER  Pi + 2    │──── 124  │
        │           │   └──────────────────────┘          │
        │           │                      │              │
   126──┐           │                      │       ┌──128 │
  ┌──────────────────┐          ┌─────────────────────────┐
  │ ΔP₁= Pi + 1 − Pi │          │ ΔP₂= Pi + 2 − Pi + 1    │
  └──────────────────┘          └─────────────────────────┘
           │                              │
           ▼◄─────────────────────────────┘
  ┌──────────────────┐
  │  S = S₀ Δt       │────── 130
  └──────────────────┘
           │
           ▼
      NON ╱ ΔP₁ ≥ S ╲ OUI            134──┐  ╱ ΔP₂ ≥ S ╲ NON
    ◄────╲    ?     ╱───────────────────►╲     ?      ╱────
          ╲────────╱                      ╲──────────╱
              │132                              │ OUI
                                                ▼
                                     ╱─────────────────────╲
                                    ╱   GENERER             ╱
                                   ╱  SIGNAL ALARME        ╱
                                  ╱───────────────────────╱
```

$\Delta P_1 = Pi + 1 - Pi$

$\Delta P_2 = Pi + 2 - Pi + 1$

$S = S_0 \Delta t$

$\Delta P_1 \geq S$ ?

$\Delta P_2 \geq S$ ?

EP 0 646 382 A1

# FIG-9

$i = 0$ —150

152— $i = 2$ — OUI — $\Delta t = \Delta t_i$ —160 — FIN

NON

154— $|P_{i+1} - P_i| > K_1 S_0 \Delta t_i$ — NON

OUI

156— $\Delta t_{i+1} = 2 \Delta t_i$

158— $i = i + 1$

$t_a = \Delta t_{i-1}$
$(\text{SI } i \leq 0, t_a = 0)$
$t_b = \Delta t_i$ —162

ALLER A **170**
(FIG.10)

EP 0 646 382 A1

FIG-10

$i = 0$  — 170

172

$i = 3$

OUI → $\Delta t = \Delta t_i$ — 186 → FIN

NON

$\Delta t_i = \dfrac{t_a + t_b}{2}$ — 174

176

$|P_{i+1} - P_i| > K_1 S_0 \Delta t_i$

NON →

182

$|P_{i+1} - P_i| < K_2 S_0 \Delta t_i$

NON → $\Delta t = \Delta t_i$ → FIN

OUI

178 — $t_a = \Delta t_i$
$t_b = t_b$

OUI → $t_a = t_a$
$t_b = \Delta t_i$ — 184

$i = i + 1$ — 180

EP 0 646 382 A1

Office européen
des brevets

# RAPPORT DE RECHERCHE EUROPEENNE

Numero de la demande

EP 94 40 2186

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | US-A-4 882 575 (KAWAHARA)<br>* le document en entier *<br>--- | 1-20 | A61M5/168 |
| X | EP-A-0 328 163 (IVAC CORP)<br>* colonne 8, ligne 45 - colonne 9, ligne 11 *<br>--- | 1 | |
| X | US-A-4 530 696 (BISERA ET AL)<br>* colonne 3, ligne 14 - ligne 42; figures *<br>--- | 1 | |
| A | DE-C-37 41 802 (B.BRAUN MELSUNGEN)<br>* colonne 3, ligne 23 - ligne 33; figure *<br>--- | 1 | |
| A | US-A-4 743 228 (BUTTERFIELD)<br>* colonne 2, ligne 5 - ligne 68 *<br>----- | 2-20 | |

DOMAINES TECHNIQUES
RECHERCHES (Int.Cl.6)

A61M

Le présent rapport a été établi pour toutes les revendications

| Lieu de la recherche | Date d'achèvement de la recherche | Examinateur |
|---|---|---|
| LA HAYE | 18 Novembre 1994 | Clarkson, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)